# EUROPEAN PATENT APPLICATION

(11) **EP 4 831 270 A1**
(43) Date of publication of application: **28.10.2026**
(21) Application number: 25171925.8
(22) Date of filing: 23.04.2025
(51) Int. Cl.: C12M 1/33, A61M 1/00, B02C 15/00, C12M 1/00

(54) **PROCESSING DEVICE AND METHOD FOR PREPARING CELLS FROM BIOLOGICAL TISSUE**

(71) Applicant: MorphoMed GmbH, 1030 Vienna (AT)
(72) Inventor: Ganz, Martin, 1030 Wien (AT)
(74) Representative: SONN Patentanwälte GmbH & Co KG

(57) **Abstract**

Processing device (1) for mechanically processing biological tissue and method for preparing cells from biological tissue using the processing device (1), the processing device (1) comprising: an inlet (4) for feeding biological tissue to the processing device (1), a stator (6) and a rotor (7) configured for rotating relative to the stator (6) about an axis of rotation (8), an outlet (5) for discharging processed biological tissue from the processing device, wherein the stator (6) comprises a stator processing face (11) and the rotor (7) comprises a rotor processing face (9) facing the stator processing face (11), wherein the rotor (7) and the stator (6) are configured to process biological tissue in a processing zone (13) between the rotor processing face (9) and the stator processing face (11) upon rotation of the rotor (7), wherein at least one feed channel (10) is provided in the rotor processing face (9), wherein the feed channel (10) extends between a radial inner end (19) and a radial outer end (20), wherein the radial inner end (19) of the at least one feed channel (10) is configured to receive biological tissue fed via the inlet (4), and wherein the feed channel (10) is closed at its radial outer end (20) towards the outer circumference of the rotor (7).

## Description

The present invention concerns a processing device for mechanically processing biological tissue, the processing device comprising: an inlet for feeding biological tissue to the processing device, a stator, a rotor configured for rotating relative to the stator about an axis of rotation, an outlet for discharging processed biological tissue from the processing device, wherein the stator comprises a stator processing face and the rotor comprises a rotor processing face facing the stator processing face, and wherein the rotor and the stator are configured to process biological tissue in a processing zone between the rotor processing face and the stator processing face upon rotation of the rotor. The present invention also concerns a method for preparing cells from biological tissue, in particular from adipose tissue, using such a processing device.

Human adipose tissue is an attractive and abundantly available source of adult stem cells applicable in regenerative medicine and tissue engineering. The adipose tissue and the derived stromal vascular fraction (SVF) and the adipose-derived stromal/stem cells (ASC) contained therein have been successfully used in clinical studies and clinical trials for treating soft tissue defects, bone defects, gastrointestinal lesions, immune disorders, neurological injuries, and cardiovascular diseases.

The addition of therapeutic cells, such as those contained in the SVF, in cell-assisted lipotransfer (CAL) can reduce postoperative atrophy in breast augmentation, increase fat graft survival, and lead to neo-vascularization inside the grafts. The interaction between cells of the grafted adipose tissue and cells of the surrounding tissue triggers repair mechanisms by releasing cytokines and growth factors.

Translation into clinics is possible according to either an autologous or an allogeneic paradigm. In both cases, devices and procedures need to be established that allow for a safe and efficient isolation of adipose tissue-derived cells that can be either implemented in a closed system during the same surgical procedure as harvesting the adipose tissue and administering the isolated cells, or under Good Manufacturing Practices for subsequent cell cultivation and expansion. Several companies developed systems aiming for a closed, sterile, safe, and reproducible cell isolation process limiting risk for contamination and unpredictability of the cell material.

WO 2017/186795 A1 discloses a non-enzymatic method and device to enrich adipose tissue with therapeutic cells.

US 4,307,846 A discloses a homogenizing apparatus for cell disruption. It includes a tube in which a rotor ("pestle member") is rotatably mounted. The diameter of the rotor is selected such that a distance between an inner surface of the tube and an outer surface of the rotor is adjusted to the cell nuclei or organelles to be isolated. The narrow spacing leads to a shearing effect, whereby cells are broken open. The rotor has a disruption portion and a feed portion. A plurality of grooves is provided in the feed portion. The depth of the grooves decreases from a maximum on the inlet side to a minimum on the outlet side. The grooves ultimately taper to the maximum outer diameter of the rotor. Inlet-side recesses serve to collect larger tissue particles, which are guided into the grooves, where they are ultimately disrupted.

An object of the present invention is to provide a method and device for mechanically processing biological tissue with improved reliability and efficacy compared to existing solutions. The invention aims to minimize cellular damage of target components of the biological tissue and preferably operate non-enzymatically to avoid the need for enzymatic digestion. In a preferred embodiment, the processing is designed to yield a population of dissociated cells or small clusters (15-20 cells) while preserving the structural integrity of the target cells (for example, having a diameter of approximately a few tens of micrometers) and at the same time enforcing a strict upper limit on the size of components (cells or clusters) that can leave the device. Structural integrity of other components than the target cells may not be preserved; adipocyte disruption may be anticipated, for example, and disintegration of extracellular matrix may also be considered acceptable or even desirable in some use-cases.

The term "biological tissue" as used herein refers broadly to any organic material derived from living organisms, including but not limited to adipose tissue, muscle, or connective tissues. While the present invention is particularly advantageous when applied to pre-fragmented biological materials such as lipoaspirate (e.g., tissue obtained via liposuction and already partially reduced in size), the scope of "biological tissue" encompasses both native, intact tissue and processed forms that may have been preconditioned or mechanically altered prior to processing. The use of this general term is intentional to ensure broad applicability across various tissue sources and preparation states, unless explicitly restricted by context. Notably, the invention is designed to process materials like lipoaspirate efficiently due to their pre-fragmented nature, but it is not limited to such applications-provided the input material is compatible with the mechanical processing capabilities of the device as described. In the exemplary application to lipoaspirate, the present disclosure helps to generate micro-fragmented fat tissue, characterized by a reduced particle size.

The processing device of the type mentioned in the outset according to the present disclosure comprises at least one feed channel provided in the rotor processing face, wherein the feed channel extends between a radial inner end and a radial outer end, wherein the radial inner end of the at least one feed channel is configured to receive biological tissue fed via the inlet of the processing device, and wherein the feed channel is closed at its radial outer end towards the outer circumference of the rotor. The feed channel being closed means that the channel itself is a dead end for the material being processed, such that biological tissue in the direction of flow from the inlet to the outlet can only exit the feed channel via the processing zone between the rotor processing face and the stator processing face. The closed-end feed channel design ensures that all tissue passing through the channel is subjected to the mechanical forces within the processing zone, leading to consistent and reproducible results.

Due to its geometry and arrangement with increasing, preferably continuously increasing, radial distance in the direction of flow, the feed channels achieve a radial transport during processing. In other words, the direction of the transport of the tissue being processed has a component pointing away from the axis of rotation. Consequently, assuming for the sake of an example, a constant rotation speed of the rotor, the material transported in the feed channel is exposed to an increasing local relative speed between rotor and stator due to the increasing distance from the axis of rotation during propagation inside the feed channel. The increasing local relative speed between the rotor and stator, as a result of the channel geometry and rotor rotation, enhances the efficiency of the mechanical processing and minimizes the required processing time. This design is also expected to reduce clogging and ensure a continuous flow of processed tissue.

While the rotation of the rotor may achieve a self-propelling effect of the device on the material being processed due to the arrangement and shape of the feed channel, this is not required and the material may preferably be pushed into the device from the inlet by an external pump or drive connected to the inlet and/or sucked through the processing device by an external pump or drive connected to the outlet. The processing zone may be flat (like a circular plate) or conical, in which case the transport in the feed channels is radially and axially at the same time. The flat configuration lends itself particularly well to miniaturization of the processing device, because it provides the greatest ratio of working volume (effectively, the volume of the processing zone) to total device volume.

The feed channel may comprise a depth, wherein the maximum depth of the feed channel may be close to the radial inner end, wherein the depth preferably decreases continuously from this maximum depth towards the radial outer end. The decreasing depth of the feed channel in the direction of flow increases the exposure of the material in the feed channel to the stator processing face. In other words, the volume in the feed channel enclosed between the rotor and the stator decreases relative to the surface of the channel in contact with the stator processing face. As the depth decreases, the volume of the feed channel diminishes while the surface area in contact with the stator processing face remains relatively constant or even increases, depending on the width of the feed channel, which may be relatively constant or increase slightly along at least the larger part of the feed channel. Toward the radial outer end, the width of the feed channel can shrink quickly in a last section of the feed channel to continuously close the feed channel. The decreasing depth may also help to prevent tissue from becoming trapped within the channel and facilitates its movement towards the processing zone.

Optionally, at least one discharge channel separate from the at least one feed channel may be provided in the rotor processing face, wherein the discharge channel extends between a radial inner end and a radial outer end, wherein the radial outer end of the at least one discharge channel is configured to discharge biological tissue to the outlet, wherein the radial inner end of the at least one discharge channel is separated from the inlet by the processing zone. The radial outer end of the discharge channel may effectively be an opening in the circumference of the rotor. The discharge channel may be arranged next to the feed channel at a distance across the processing zone. The relative positioning of the discharge channel to the feed channel defines the processing path length - the distance travelled by the tissue within the processing zone. By adjusting the processing path length, the degree of mechanical processing can be precisely controlled. A shorter processing path length results in less intense processing, while a longer processing path length allows for more thorough disruption or separation of the tissue components. This ability to control the processing path length allows for tailored processing of heterogeneous tissues, enabling selective disruption of certain components while preserving the integrity of others. In particular, the discharge channel allows to control the processing path length independently from the radial location where the tissue enters the processing zone (due to its size or other properties). Due to the discharge channel, not all material is exposed to the maximum relative speed in the processing zone, because once it escapes the processing zone and enters the discharge channel, it can leave the rotor without entering the processing zone again.

The discharge channel may comprise a depth, wherein the maximum depth of the discharge channel may be close to the radial outer end, wherein the depth preferably decreases continuously from this maximum depth towards the radial inner end. The increasing depth facilitates transport and takes into account that the discharge channel collects material at different radial positions at the same time, which collectively needs to be transported radially outward. For example, the cross section of the discharge channel may increase toward the outer end at the same rate that the cross section of the feed channel decreases (see the discussion about its depth above). This increase may partially be achieved not only by providing an increasing depth but also by providing an increasing width of the discharge channel toward the radial outer end. In this instance, the discharge channel may split between the radial inner end and the radial outer end for structural reasons, such that the number of radial outer ends is greater than the number of radial inner ends.

The rotor may optionally comprise at least two feed channels and at least two discharge channels, wherein the feed channels and the discharge channels are arranged in an alternating pattern in a circumferential direction of the rotor processing face. Such a multi-channel arrangement can increase the throughput of the device without deteriorating the processing performance. It is particularly useful to compensate for miniaturization of the device. For example, more than half of the surface area of the rotor processing surface may be used by the channels, including feed channels and discharge channels.

In one embodiment, the stator comprises a through-hole for connecting the at least one feed channel to the inlet. In other words, the biological tissue entering through the inlet traverses the stator through the through-hole before it enters the at least one feed channel of the rotor. Generally, a through-hole may also be provided in the rotor; the arrangement of the through-hole in the stator facilitates the mounting and bearing of the rotor as the rotor can omit a second through-hole.

In this embodiment, an opening of the inlet toward the rotor may optionally be arranged eccentrically, in particular at a distance of at least 1 millimeter (mm) from the axis of rotation. One advantage of the eccentric arrangement is that the entire opening can be swiped by the feed channels, avoiding a dead spot at the axis of rotation and dead zones forming within the inlet or directly behind the inlet where unprocessed tissue can collect and clog the inlet. Moreover, the feed channels may be separated by ribs extending right to the axis of rotation, i.e. there is no fluid connection between two or more feed channels across the center of the rotor in this embodiment.

In another embodiment, at least one processing groove may be provided in the stator processing face, wherein the processing groove preferably extends between a radial inner end and a radial outer end. The radial inner end may be at the edge of a through-hole formed in the stator, if any. The radial outer end may be at the circumference of the stator, or opposite the circumference of the rotor, if the rotor has a smaller diameter than the stator. The processing groove increases the mechanical stress on the tissue transported gliding along the stator processing face. It may also serve to protect tissue components below a certain size by allowing those components to enter the processing groove, thus escaping the processing zone at least temporarily, for example during passage from a feed channel to a discharge channel. The number of processing grooves along a full round-trip along the stator processing face at a given radius may be higher in a radial outer section than in a radial inner section of the stator processing face to increase the efficiency in the radial outer section and avoid leakage in the radial inner section. Consequently, the processed material dragged along in the feed channel of the rotor interacts with a larger number of processing grooves per round (and -hence - time unit) of the rotor movement when it has travelled in the rotor to a radial outer section compared to when it arrives first at a radial inner section after entering the feed channel. The processing grooves may have right-angled edges relative to the stator processing face; for example, they may have a rectangular profile. The width of the processing grooves may be chosen to fit the material to be processed, in particular the target components to be spared. The profile of the processing grooves is preferably larger than the target components.

In this embodiment, a longitudinal channel axis of the at least one feed channel may have a spiral shape, wherein the processing groove has a longitudinal groove axis, wherein a projection of the longitudinal groove axis onto the longitudinal channel axis forms an angle, in particular a right angle, in at least one intersection. The spiral shape of the feed channel can contribute to a transport of the biological tissue in the feed channel as it exerts a radial force, effectively pushing the tissue radially outward through the feed channel. The spiral may be an Archimedean spiral. In an alternative embodiment, not the longitudinal channel axis, but the radial inner channel edge of the at least one feed channel may be the reference having a spiral shape and forming an angle with the projected groove axis. In this case, the radial outer edge would not have the same spiral shape, as the width of the channel generally varies. In both cases, the spiral shape generally does not begin at the center of the rotor at the axis of rotation, but at a finite, non-zero feed channel offset radius. Optionally, a longitudinal channel axis (or a radial inner channel edge) of the at least one discharge channel may also have a spiral shape similar to that of the feed channel. Here, the spiral shape generally begins even further away from the axis of rotation, at a discharge channel offset radius that is greater than the feed channel offset radius by at least the radius of the through hole in the stator, if any.

The method of the type mentioned in the outset according to the present disclosure comprises the following steps:
- providing a processing device according to one or more of the variations and embodiments discussed above;
- continuously feeding biological tissue to the inlet of the processing device;
- processing the biological tissue by rotating the rotor against the stator, in particular thereby mechanically separating cells and/or cell aggregates within biological tissue;
- withdrawing processed biological tissue from the outlet of the processing device.

The method shares the advantages of the device discussed above. As becomes even more apparent from the method, the disclosed device is designed for continuous (rather than batch) processing of biological tissue, which has many advantages depending on the actual use of the device and for example allows for rapid re-purposing of the processed biological tissue.

Optionally, the method may use a device having a discharge channel separate from the at least one feed channel provided in the rotor processing face, and comprise the further steps of transporting biological tissue from the feed channels to at least one discharge channel upon processing, wherein the discharge channel is connected to the outlet. The advantages of the discharge channels have been discussed above in relation to the processing device. These advantages also apply to the method using that embodiment of the device.

In another variation, the method may use a device having at least one processing groove provided in the stator processing face, where processing the biological tissue comprises temporarily capturing biological tissue in the at least one processing groove upon rotation of the rotor. The advantages of the processing grooves have been discussed above in relation to the processing device. These advantages also apply to the method using that embodiment of the device.

Optionally, the biological tissue may not be processed alone, but a diluent solution may be fed to the inlet of the processing device in conjunction with the biological tissue. The diluent solution helps to prevent clogging during mechanical processing. Various physiological saline/buffer solutions can be used as a dilution solution, for example: isotonic saline ("saline"), PBS ("phosphate-buffered saline"), and various full electrolyte solutions or various buffer systems.

The feed rate entering the inlet may be below 10 milliliters per second (ml/s), preferably 1-4 ml/s. The rotation speed during processing may be between 1000 and 2000 rounds per minute (rpm), preferably approximately 1500 rpm. Rotation speeds in these ranges are particularly suitable for processing lipoaspirate at the current dimensions of the device (rotor diameter of 5-6 centimeters). Other parameters may be better suited for different biological tissue, or generally different material, and/or different dimensions of the processing device.

The invention will be defined in more detail below by means of exemplary embodiments, to which it is not to be limited to, however, and with reference to the drawings. In detail:
Fig. 1 shows a processing device for mechanically processing biological tissue according to a first embodiment;
Fig. 2 shows an exploded view of the device according to Fig. 1, revealing its internal construction;
Fig. 3 presents a side view of the device according to Fig. 1, focusing on the outlet side;
Fig. 4 shows a section view along the lines IV-IV in Fig. 3, providing further insight into the internal arrangement of the components and illustrating the processing zone;
Fig. 5 depicts a top view of the rotor processing face of the device according to Fig. 1;
Fig. 6 shows a corresponding view of the stator processing face according to Fig. 1;
Fig. 7 shows a top view of the stator exterior according to Fig. 1, revealing the rotor through a through-hole in the stator;
Fig. 8 shows a top view similar to Fig. 7, with the stator removed, illustrating the rotor in a surrounding collection chamber and transport of processed tissue towards the outlet;
Figs. 9-11 show a second embodiment of the processing device with modifications to the rotor and stator processing faces, showing views similar to Figs. 5-7 for the first embodiment, respectively;
Fig. 12 shows a third embodiment of the processing device similar to the second embodiment, without paddles on the rotor.

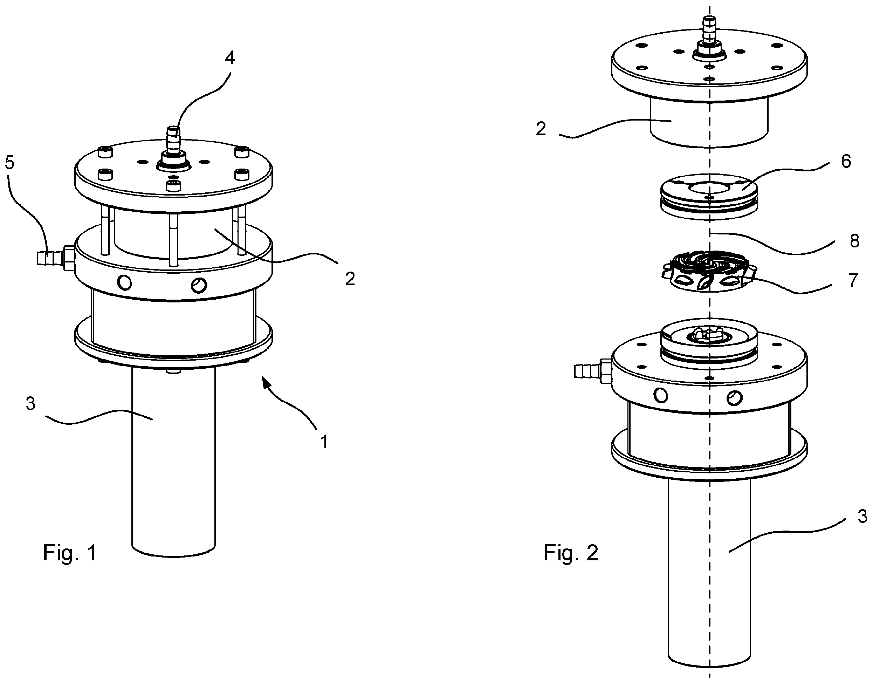

Fig. 1 shows a first embodiment of a processing device 1 according to the present disclosure. The processing device 1 is designed for mechanically processing biological tissue. It comprises a housing 2 and an electric motor 3. The housing 2 has an inlet 4 for feeding tissue and an outlet 5 for discharging processed tissue.

Fig. 2 shows an exploded view of the processing device 1 according to Fig. 1. It reveals a stator 6 and a rotor 7 of the processing device 1 that in operation are arranged inside the housing 2. The rotor 7 is configured for relative rotation about an axis 8. The rotor 7 is coupled to the electric motor 3 for rotation drive.

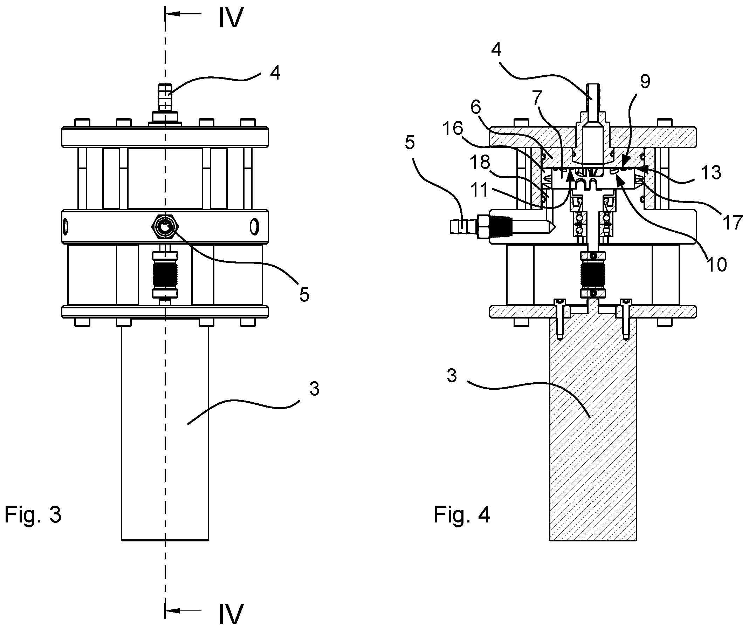

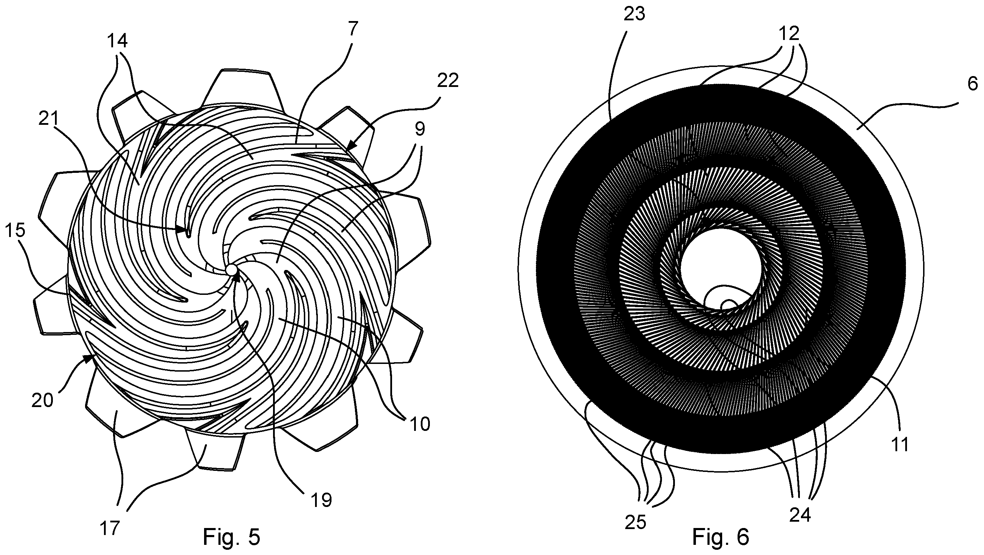

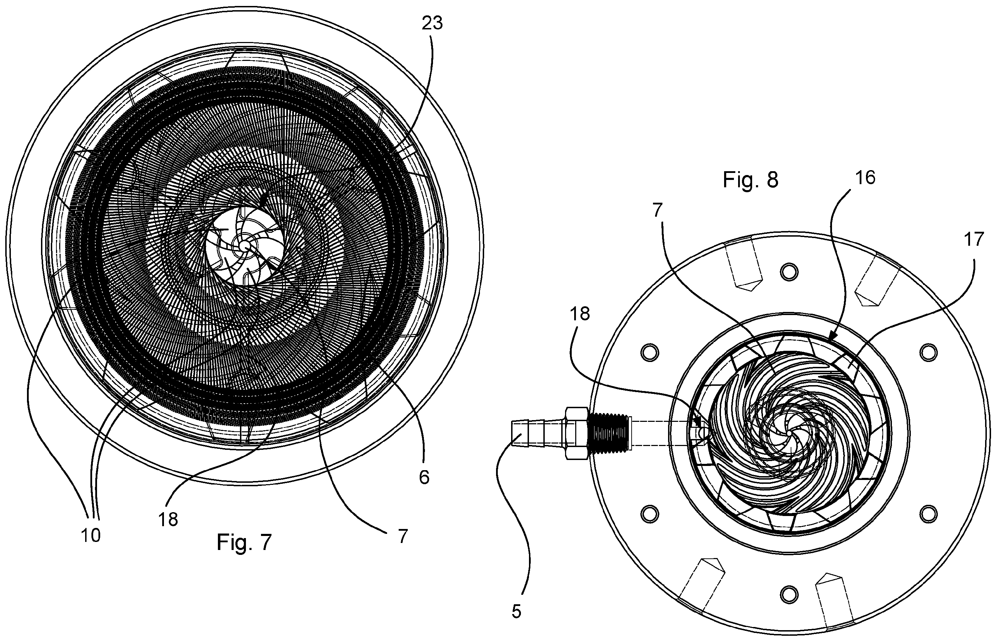

The movement of tissue through the assembled processing device 1 can be understood from Figs. 3 and 4. The biological tissue is fed through the inlet 4. From there it gets in contact with the rotor processing face 9 (see Fig. 5). There, it is pushed along feed channels 10 while the rotor 7 is rotated. Through the rotation, the tissue is moved past and along the stator processing face 11 and across its processing grooves 12 (see Fig. 6), thereby continuously being processed. Once the tissue components have become small enough to be captured in one of the processing grooves 12 or they otherwise fit into a processing zone 13 between the rotor 7 and the stator 6, they move from the feed channels 10 into discharge channels 14 (see Fig. 5), effectively finishing their processing. More precisely, the processing zone 13 is the volume between the flat parts of the rotor processing face 9 between the feed channels 10 and discharge channels 14 on one side and the stator processing face 11 on the opposite side. Once in the discharge channels 14, the processed tissue continues being pushed radially outwards toward and over the edge 15 of the rotor 7 into the collection chamber 16. In the collection chamber 16, the processed tissue is moved by means of the paddles 17 on the rotor 7 until it is sucked out through a drain 18 to the outlet 5 (see also Fig. 8).

In total, five feed channels 10 are provided in the rotor processing face 9, extending from a radial inner end 19 (receiving tissue from the inlet 4) to a radial outer end 20. The feed channels 10 follow a spiral shape with their longitudinal axis. The feed channels 10 are closed at their radial outer ends 20, retaining the tissue within the feed channels 10 until it is processed sufficiently for entering and traversing the processing zone 13 at any position along the spiral shape. The processing device 1 functions by rotating the rotor 7, thereby processing tissue while in the feed channels 10 and until it passes through the processing zone 13. The feed channels 10 have a depth, with the maximum depth near the radial inner end 19. This depth preferably decreases continuously toward the radial outer end 20, continuously increasing tissue exposure during processing.

Similarly, five corresponding discharge channels 14, separate from the feed channels 10, are provided in the rotor processing face 9. The discharge channels 14 extend from radial inner ends 21 to radial outer ends 22. The discharge channels 14 empty into the collection chamber 16 around the rotor 7 and from there finally to the drain 18 and to the outlet 5. The discharge channels 14 have a depth, with the maximum depth near the radial outer end 22, hence increasing continuously from the radial inner ends 21 toward the radial outer ends 22. The feed channels 10 and the discharge channels 14 are arranged in an alternating pattern around the rotor processing face 9. The radial inner ends 21 (beginnings) of the discharge channels 14 are separated from the inlet 4 by the processing zone 13. As shown in Fig. 7, the stator 6 comprises a through-hole 23 connecting the inlet 4 to the radial inner ends 19 (beginnings) of the feed channels 10.

The stator processing face 11 shown in Fig. 6 comprises a manifold of processing grooves 12 extending between radial inner ends 24 and radial outer ends 25. The processing grooves 12 of the first embodiment are straight, each defining a longitudinal groove axis. The projections of these axes are at a right angle with the feed channel axes of the feed channels 10 in the rotor 7 (see Fig. 7 with hidden structures indicated in dashed lines).

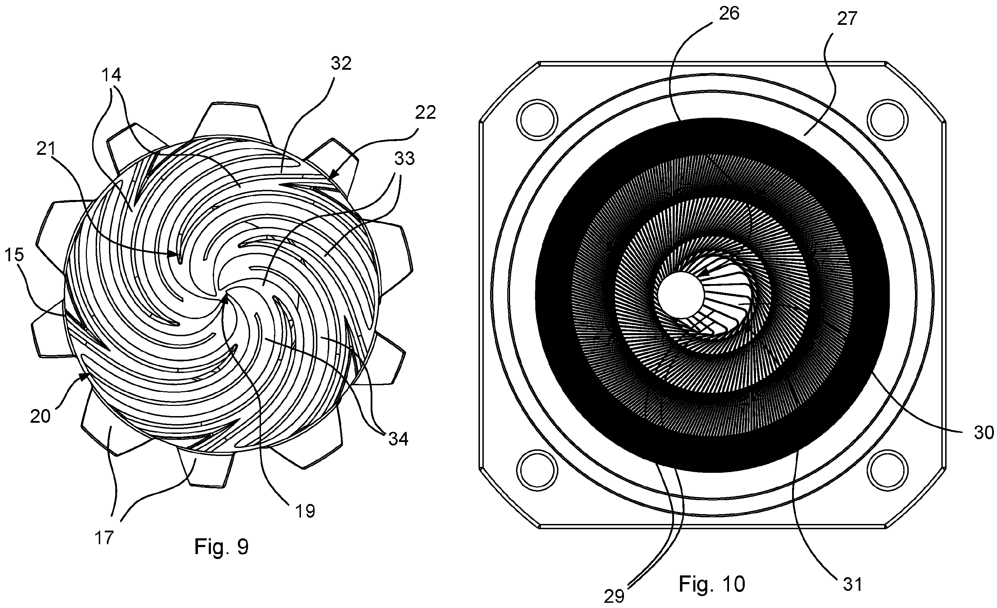

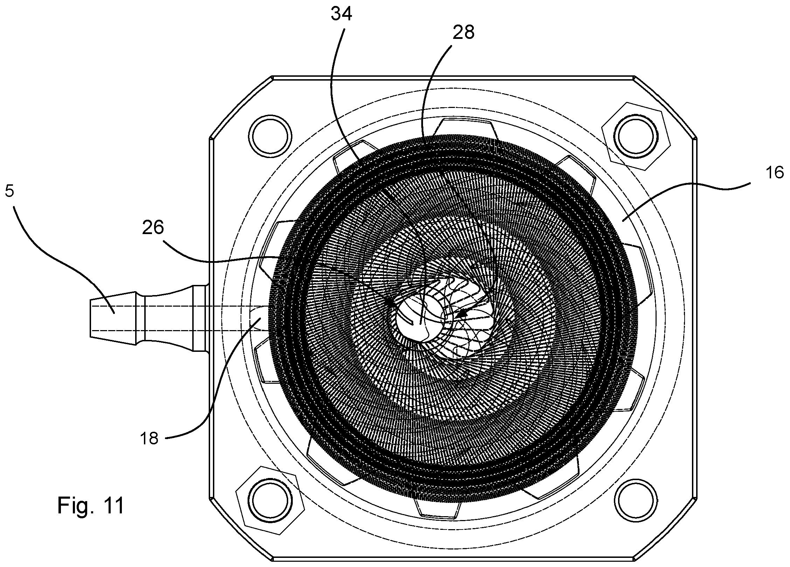

The second embodiment of the processing device shown in Figs. 9-11 differs from the first embodiment mainly in that the through-hole 26 in the stator 27 is arranged eccentrically, in particular at a distance of approximately 4 mm from the axis of rotation 28 (see Fig. 11). The processing grooves 29 in the stator processing face 30 in a central region 31 at radii lower than the centre of the through-hole 26 fan out from the edge of the through-hole 26 across the central region 31 and have a bend at the limit of the central region 31, after which they continue straight, similar to the first embodiment (see Fig. 10). The rotor 32 is the same as in the first embodiment, including the rotor processing face 33 (see Fig. 9). The eccentric arrangement of the through-hole 26 has the effect that the fed tissue enters one or two of the five feed channels 34 at any given moment. The edge of the through-hole 26 facing the rotor 32 is swiped by the feed channels' 34 edges, avoiding dead zones in this part of the processing device. Other elements of this embodiment are indicated with the same reference signs as used for the first embodiment and it is referred to the above description, which applies analogously, to avoid repetitions.

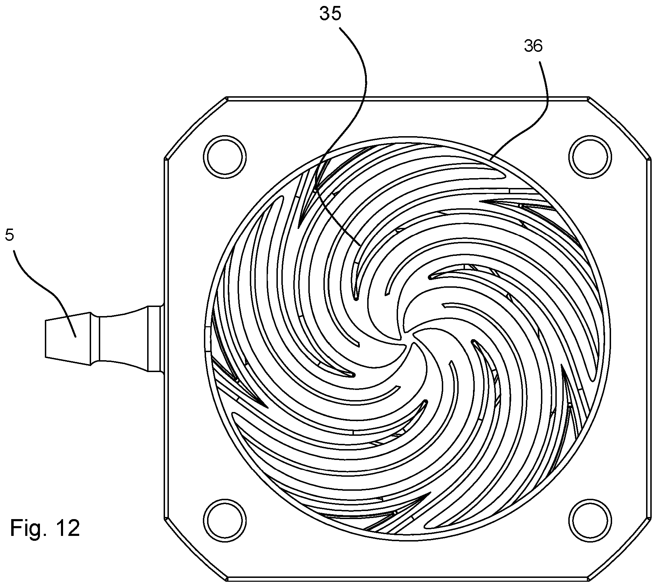

The third embodiment shown in Fig. 12 differs from the first and second embodiment in that it uses a rotor 35 without paddles, i.e. that essentially fills the collection chamber 36.

The devices discussed above are suitable for use in a method for preparing cells from biological tissue, particularly adipose tissue. The method involves continuously feeding a diluent solution comprising biological tissue to the inlet at a feed rate of 1-4 ml/s, processing the tissue by rotating the rotor against the stator (mechanically separating cells/aggregates), and withdrawing the processed tissue from the outlet.

## Claims

1. Processing device (1) for mechanically processing biological tissue, the processing device (1) comprising:
- an inlet (4) for feeding biological tissue to the processing device (1),
- a stator (6) and a rotor (7) configured for rotating relative to the stator (6) about an axis of rotation (8),
- an outlet (5) for discharging processed biological tissue from the processing device,
wherein the stator (6) comprises a stator processing face (11) and the rotor (7) comprises a rotor processing face (9) facing the stator processing face (11),
wherein the rotor (7) and the stator (6) are configured to process biological tissue in a processing zone (13) between the rotor processing face (9) and the stator processing face (11) upon rotation of the rotor (7),
***characterized in that*** at least one feed channel (10) is provided in the rotor processing face (9), wherein the feed channel (10) extends between a radial inner end (19) and a radial outer end (20), wherein the radial inner end (19) of the at least one feed channel (10) is configured to receive biological tissue fed via the inlet (4), and wherein the feed channel (10) is closed at its radial outer end (20) towards the outer circumference of the rotor (7).

2. Processing device (1) according to claim 1, **characterized in that** the feed channel (10) comprises a depth, wherein the maximum depth of the feed channel (10) is close to the radial inner end (19), wherein the depth preferably decreases continuously from this maximum depth towards the radial outer end (20).

3. Processing device (1) according to claim 1 or 2, **characterized in that** at least one discharge channel (14) separate from the at least one feed channel (10) is provided in the rotor processing face (9), wherein the discharge channel (14) extends between a radial inner end (21) and a radial outer end (22), wherein the radial outer end (22) of the at least one discharge channel (14) is configured to discharge biological tissue to the outlet (5), wherein the radial inner end (21) of the at least one discharge channel (14) is separated from the inlet (4) by the processing zone (13).

4. Processing device (1) according to claim 3, **characterized in that** the discharge channel (14) comprises a depth, wherein the maximum depth of the discharge channel (14) is close to the radial outer end (22), wherein the depth preferably decreases continuously from this maximum depth towards the radial inner end (21).

5. Processing device (1) according to claim 3 or 4, **characterized in that** the rotor (7) comprises at least two feed channels (10) and at least two discharge channels (14), wherein the feed channels (10) and the discharge channels (14) are arranged in an alternating pattern in a circumferential direction of the rotor processing face (9).

6. Processing device (1) according to any one of claims 1 to 5, wherein the stator (6) comprises a through-hole (23) for connecting the at least one feed channel (10) to the inlet (4).

7. Processing device (1) according to claim 6, **characterized in that** an opening of the through-hole (26) toward the rotor (32) is arranged eccentrically, in particular at a distance of at least 1 mm from the axis of rotation (28).

8. Processing device (1) according to any one of claims 1 to 7, **characterized in that** at least one processing groove (12) is provided in the stator processing face (11), wherein the processing groove (12) preferably extends between a radial inner end and a radial outer end.

9. Processing device (1) according to claim 8, wherein a longitudinal channel axis of the at least one feed channel (10) has a spiral shape, wherein the processing groove (12) has a longitudinal groove axis, wherein a projection of the longitudinal groove axis onto the longitudinal channel axis forms an angle, in particular a right angle, in at least one intersection.

10. Method for preparing cells from biological tissue, in particular from adipose tissue, the method comprising the following steps:
- providing a processing device (1) according to any one of claims 1 to 9;
- continuously feeding biological tissue to the inlet (4) of the processing device (1);
- processing the biological tissue by rotating the rotor (7) against the stator (6), in particular thereby mechanically separating cells and/or cell aggregates within the biological tissue;
- withdrawing processed biological tissue from the outlet (5) of the processing device (1).

11. Method according to claim 10, **characterized by** the further steps:
- transporting biological tissue from the feed channels (10) to at least one discharge channel (14) upon processing, wherein the discharge channel (14) is connected to the outlet (5).

12. Method according to claim 10 or 11 with a processing device (1) according to claim 8 or 9, **characterized in that** processing the biological tissue comprises:
- temporarily capturing biological tissue in the at least one processing groove (12) upon rotation of the rotor (7).

13. Method according to any one of claims 10 to 12, **characterized in that** a diluent solution is fed to the inlet (4) in conjunction with the biological tissue.

14. Method according to any one of claims 10 to 13, **characterized in that** the feed rate entering the inlet (4) is below 10 ml/s, preferably 1-4 ml/s.
